# EUROPÄISCHE PATENTSCHRIFT

(11) **EP 1 917 232 B1**
(45) Veröffentlichungstag und Bekanntmachung des Hinweises auf die Patenterteilung: **14.09.2011**
(21) Anmeldenummer: 06778022.1
(22) Anmeldetag: 27.07.2006
(51) Int. Cl.: C07C 209/48, C07C 211/27

(54) **VERFAHREN ZUR HERSTELLUNG VON XYLYLENDIAMIN DURCH KONTINUIERLICHE HYDRIERUNG VON PHTHALODINITRIL**
PROCESS FOR PREPARING XYLYLENEDIAMINE BY CONTINUOUS HYDROGENATION OF PHTHALODINITRILE
PROCEDE POUR PREPARER DE LA DIAMINE DE XYLYLENE PAR HYDRATATION CONTINUE DE PHTHALODINITRILE

(30) Priorität: 02.08.2005 DE 102005036222; 07.12.2005 DE 102005058417
(43) Veröffentlichungstag der Anmeldung: 07.05.2008
(73) Patentinhaber: BASF SE, 67056 Ludwigshafen (DE)
(72) Erfinder: HUGO, Randolf, 67246 Dirmstein (DE); DAHMEN, Kirsten, 68161 Mannheim (DE); HUBER, Sabine, 67117 Limburgerhof (DE)
(86) Internationale Anmeldenummer: PCT/EP2006/064731
(87) Internationale Veröffentlichungsnummer: WO 2007/014901

(56) Entgegenhaltungen:
- WO-A-2005/026099

## Beschreibung

Die vorliegende Erfindung betrifft ein Verfahren zur Herstellung von Xylylendiamin durch kontinuierliche Hydrierung von Phthalodinitril an einem Heterogenkatalysator in Gegenwart von flüssigem Ammoniak in einem Reaktor.

Xylylendiamin (Bis(aminomethyl)benzol) ist ein nützlicher Ausgangsstoff, z.B. für die Synthese von Polyamiden, Epoxyhärtern oder als Zwischenstufe zur Herstellung von Isocyanaten.

Die Bezeichnung "Xylylendiamin" (XDA) umfasst die drei Isomere ortho-Xylylendiamin, meta-Xylylendiamin (MXDA) und para-Xylylendiamin.

Der Begriff "Phthalodinitril" (PDN) umfasst die drei Isomere 1,2-Dicyanbenzol = o-Phthalodinitril, 1,3-Dicyanbenzol = Isophthalodinitril = IPDN und 1,4-Dicyanbenzol = Terephthalodinitril.

Die Phthalodinitrile sind Feststoffe (z.B. schmilzt Isophthalodinitril (IPDN) bei 161°C) und weisen relativ schlechte Löslichkeiten in organischen Lösungsmitteln auf.

Die zweistufige Synthese von Xylylendiamin durch Ammonoxidation von Xylol und anschließender Hydrierung des erhaltenen Phthalodinitrils ist bekannt.

Unumgesetzte Dinitrile lassen sich nur sehr schwer vom XDA destillativ trennen.

US-A-4,482,741 (UOP Inc.) beschreibt die Hydrierung von PDN in Gegenwart von Ammoniak, einem spezifischen Katalysator und XDA als Lösungsmittel.

In MXDA beträgt die Löslichkeit von IPDN bei 70°C ca. 20 Gew.-%.

EP-A2-1 193 247 und EP-A1-1 279 661 (beide Mitsubishi Gas Chem. Comp.) betreffen ein Verfahren zur Reinigung von Isophthalodinitril (IPDN) bzw. ein Verfahren zur Herstellung von reinem XDA.

EP-A2-1 193 244 (Mitsubishi Gas Chem. Comp.) beschreibt ein Verfahren zur Herstellung von XDA durch Hydrierung von Phthalodinitril, welches in einer vorherigen Stufe durch Ammonoxidation von Xylol synthetisiert wird, wobei das dampfförmige Produkt der Ammonoxidationsstufe direkt mit einem flüssigen organischen Lösungsmittel in Kontakt gebracht wird (Quench) und die erhaltene Quenchlösung oder-suspension der Hydrierung zugeführt wird.

Bevorzugte organische Lösungsmittel sind C₆-C₁₂ aromatische Kohlenwasserstoffe, wie Xylol und Pseudocumol (Spalte 6, Absatz [0027] und [0028]).

US-A-3,069,469 (California Research Corp.) lehrt als Lösungsmittel zur Hydrierung von aromatischen Nitrilen, wie PDN, aromatische Kohlenwasserstoffe, Xylol, Dioxan und aliphatische Alkohole.

DE-A-21 64 169 (Mitsubishi Gas Chemical Co., Inc.) beschreibt auf Seite 6, letzter Absatz, die Hydrierung von IPDN zu meta-XDA in Gegenwart eines Ni- und/oder Co-Katalysators in Ammoniak als Lösungsmittel.

GB-A-852,972 (Äquivalent: DE-A-11 19 285) (BASF AG) offenbart die Verwendung von Ammoniak und XDA als Lösungsmittel in der Hydrierung von PDN. Die Herstellung der Edukt-Lösung ausgehend von festem PDN erfolgt in einem Extraschritt in einem separaten Gefäß (vgl. Seite 2, Zeilen 119-120).

JP-A-2003-327563 (Mitsubishi Gas Chem. Co., Inc.) betrifft ein Verfahren zur Festbett-Hydrierung von aromatischen Dinitrilen, die als 1-10 Gew.-%ige Lösungen eingesetzt werden.

Die sechs Patentanmeldungen WO-A-05/028417, WO-A-05/026102, WO-A-05/026103, WO-A-05/026104, WO-A-05/026100 und WO-A-05/026101 (BASF AG) betreffen jeweils Verfahren zur Herstellung von XDA.

Bei den unterschiedlichen Verfahren zur Herstellung von Phthalodinitril fällt dieses als Feststoff oder gelöst in einem Lösungsmittel, z.B. Pseudocumol, oder als Schmelze an. Die Handhabung von Feststoffen ist üblicherweise schwierig und umständlich. Die Weiterverarbeitung in einem Lösungsmittel erfordert wegen der geringen Löslichkeit von Phthalodinitril in Lösungsmitteln, wie o-Xylol, m-Xylol, p-Xylol, Pseudocumol, Mesitylen, Ethylbenzol oder Methylpyridin, sehr große Lösungsmittelmengen, die nach der Hydrierung in der Regel destillativ abgetrennt werden müssen, was - entsprechend den großen Mengenströmen - große Apparate und einen hohen Energieaufwand erfordert. Alternativ ist eine Extraktion des PDNs mit Wasser mit anschließender Destillation möglich. Auch hier ist der Energieaufwand groß, da das Wasser abdestilliert und das Lösungsmittel - zumindest im Teilstrom - regeneriert werden muss.

WO-A-05/026098 (BASF AG) betrifft ein Verfahren zur Herstellung von XDA durch kontinuierliche Hydrierung von Phthalodinitril an einem Heterogenkatalysator in Gegenwart von flüssigem Ammoniak in einem Reaktor, bei dem ein Teil des Reaktoraustrags als flüssiger Umlaufstrom kontinuierlich zum Reaktoreingang zurückgeführt wird (Kreislauffahrweise), in dem mittels einer Mischeinrichtung ein Strom einer Phthalodinitrilschmelze flüssig in den Umlaufstrom um den Hydrierreaktor zugefahren wird, wobei der Phthalodinitril-Umsatz im Reaktor bei einfachem Durchgang größer 99 % beträgt, und der Umlaufstrom zu größer 93 Gew.-% aus flüssigem Ammoniak und Xylylendiamin besteht und kein weiteres Lösungsmittel für Phthalodinitril enthält.

WO-A-051026099 (BASF AG) betrifft ein Verfahren zur Herstellung von XDA durch kontinuierliche Hydrierung von PDN an einem Heterogenkatalysator in Gegenwart von flüssigem Ammoniak in einem Reaktor, wobei mittels einer Mischeinrichtung ein Strom einer Phthalodinitrilschmelze flüssig mit einem Strom von flüssigem Ammoniak gemischt wird und die flüssige Mischung in den Hydrierreaktor gefahren wird.

Wegen der hohen Schmelzpunkte lassen sich Phthalodinitrile in geschmolzener Form nur schwer und unter erheblichem Aufwand für eine Schutzbeheizung handhaben. Zur Förderung gegen Hochdruck wie in der Verfahrensweise gemäß o.g. WO-A-05/026098 erforderlich, kommen spezielle Pumpen zum Einsatz, z.B. beheizbare Hochdruckmembranpumpen, die einerseits sehr störanfällig und andererseits sehr teuer sind. Das Verfahren gemäß WO-A-051026098 hat aber den Vorteil, dass nur eine verhältnismäßig kleine Menge an flüssigem Ammoniak aufgearbeitet werden muss, nämlich die Menge, die mit dem Reaktionsprodukt Xylylendiamin aus der Reaktionsstufe zur Aufarbeitung ausgetragen wird. Die Ammoniakmenge im Reaktor kann mittels entsprechend groß gewähltem Kreislauf trotz kleinem Frisch-Amrponiakstrom so gewählt werden, dass die Reaktion unter optimalen Bedingungen ablaufen kann (z.B. Menge an NH₃ in mol pro mol eingesetztes Phthalodinitril).

Demgegenüber ist bei einer Verfahrensweise analog o.g. WO-A-05/026099 zur Förderung der Phthalodinitrilschmelze lediglich eine beheizbare Pumpe erforderlich, die die Schmelze in den Mischbehälter fördern muss, der bei deutlich niedrigerem Druck, z.B. 20 bis 40 bar betrieben wird. Hierzu kann z.B. eine kostengünstige und wenig störanfällige mehrstufige Kreiselpumpe zum Einsatz kommen. Zur Förderung der Lösung auf Reaktordruck (z.B. 200 bar) ist zwar eine zusätzliche Pumpe erforderlich, jedoch ist die Handhabung der Lösung von Phthalodinitril in Ammoniak deutlich einfacher und die Anforderungen an die Schutzbeheizung sind erheblich geringer. Entsprechend kann eine weniger teure Pumpe eingesetzt werden, die bei der tieferen Betriebstemperatur weniger störanfällig ist. Insbesondere bei Abstellungen oder Betriebsstörungen, z.B. auch in anderen Anlagenteilen, lässt sich die Lösung besser handhaben als die Schmelze. Es besteht jedoch der Nachteil, dass die Löslichkeit von Phthalodinitril in Ammoniak begrenzt und von der Temperatur abhängig ist. Bei niedriger Temperatur ist die erreichbare Phthalodinitrilkonzentration in Ammoniak klein, aber der Lösebehälter kann bei niedrigem Druck betrieben werden, was auch mit entsprechend niedrigen Anforderungen an die Schmelzepumpe einhergeht. Damit muss aber auch eine große Menge an Ammoniak nach der Hydrierung des Phthalodinitrils durch Druckdestillation wieder aufgearbeitet und zurückgewonnen werden. Steigert man die Temperatur im Lösebehälter, so lässt sich eine größere Phthalodinitrilkonzentration einstellen und entsprechend weniger Ammoniak muss aufgearbeitet werden. Andererseits steigt damit der Druck im Lösebehälter und die Anforderungen für die Schmelzepumpe und die Schutzbeheizung steigen. Die Apparate, Maschinen und Einrichtungen werden teurer und störanfälliger.

Der vorliegenden Erfindung lag die Aufgabe zugrunde, ein verbessertes Löseverfahren zur Lösung von Phthalodinitril aufzufinden, das möglichst die Vorteile der beiden geschilderten Verfahrensvarianten analog WO-A-05/026098 und WO-A-05/026099 vereint und die Nachteile unterdrückt.

Demgemäß wurde ein Verfahren zur Herstellung von Xylylendiamin durch kontinuierliche Hydrierung von Phthalodinitril an einem Heterogenkatalysator in Gegenwart von flüssigem Ammoniak in einem Reaktor, wobei ein Teil des Reaktoraustrags als flüssiger Umlaufstrom kontinuierlich zum Reaktoreingang zurückgeführt wird (Umlaufkreisfahrweise), gefunden, welches dadurch gekennzeichnet ist, dass mittels einer Mischeinrichtung Phthalodinitril als Schmelze oder in fester Form mit einem Strom von flüssigem Ammoniak (Strom a) und
einem weiteren Strom, der zumindest als Teilstrom aus dem Umlaufstrom um den Hydrierreaktor abgezogen wird (Strom b),
oder einer Mischung aus den Strömen a und b gemischt wird und die resultierende flüssige Mischung in den Hydrierreaktor gefahren wird, so dass den Umlaufstrom mindestens teilweise nicht direkt zum Reaktor zurückgeführt
wird, sondern in direkt über eine Mischeinrichtung.

In einer besonderen Ausgestaltung des erfindungsgemäßen Verfahrens wird als Hydrierreaktor, z.B. anstelle eines einzelnen Hydrierreaktors, eine Hydrierreaktorkaskade eingesetzt.

Die Reaktorkaskade besteht dann aus 2, 3, 4 oder mehr hintereinander geschalteten Hydrierreaktoren. Gemäß der Umlaufkreisfahrweise wird ein Teil eines Reaktoraustrags (z.B. des letzten Reaktors) als flüssiger Umlaufstrom kontinuierlich zu einem Reaktoreingang (z.B. des ersten Reaktors) zurückgeführt.

Die Mischeinrichtung wird bevorzugt bei einem Druck betrieben, welcher kleiner ist als der Druck im Reaktor.

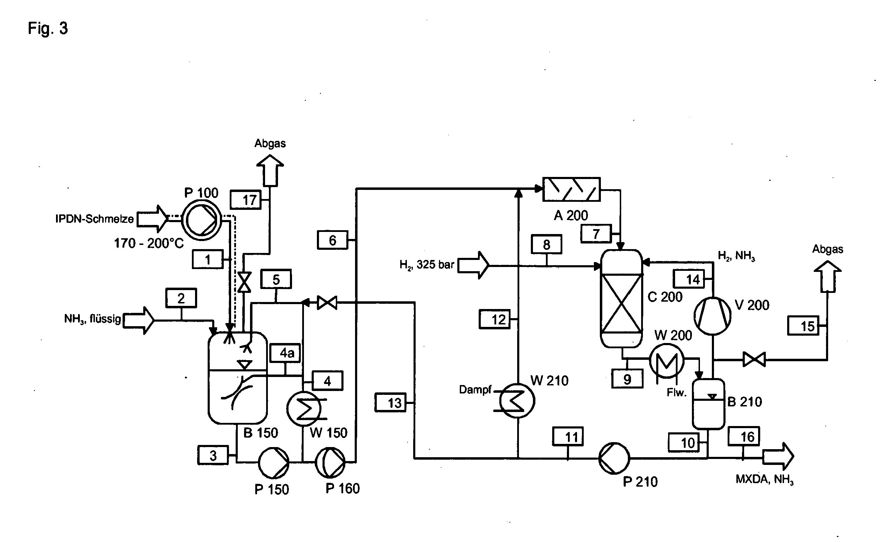

Der Umlaufstrom um den Reaktor (z.B. Strom [11] in Abb. 3) wird somit mindestens teilweise nicht direkt (z.B. als Strom [12] in Abb. 3) zum Reaktor zurückgeführt, sondern indirekt (z.B. als Strom [13] in Abb. 3) über die Mischeinrichtung. Der Reaktorumlaufstrom besteht, da praktisch Vollumsatz gefahren und bevorzugt kein weiteres Lösungsmittel verwendet wird, aus einer flüssigen Mischung von Xylylendiamin, Ammoniak und ggf. Nebenprodukten bzw. Nebenkomponenten.

Bevorzugt findet das erfindungsgemäße Verfahren Anwendung zur Herstellung von meta-Xylylendiamin (MXDA) durch Hydrierung von Isophthalodinitril (IPDN), welches insbesondere in einer vorherigen Stufe durch Ammonoxidation von meta-Xylol synthetisiert wurde.

Das geschmolzene Phthalodinitril kann beispielsweise aus einem einer Ammonoxidation nachgeschalteten Quench, einer Eindampfstufe oder einer Destillationskolonne kommen, wobei das Phthalodinitril z.B. jeweils als Schmelze über Sumpf (oder Seitenabzug) dieser thermischen Trennapparate abgetrennt wird, wie z.B. in WO-A-05/026103 (BASF AG) beschrieben.

Alternativ kann im erfindungsgemäßen Verfahren auch aufgeschmolzenes, zuvor als Feststoff vorliegendes PDN eingesetzt werden. Das Aufschmelzen kann z.B. mittels eines Extruders erfolgen.

Eine weitere Möglichkeit besteht darin, festes Phthalodinitril, das z.B. in Form von Pulver, Schuppen oder Pellets vorliegt, kontinuierlich, diskontinuierlich oder halbkontinuierlich in einem durchmischten Behälter (z.B. Rührbehälter), Rührkessel oder sonstigen Mischeinrichtung mit den beiden anderen Strömen aus Ammoniak (Strom a) und dem Teilstrom oder Gesamtstrom (Strom b) aus dem Reaktorumlauf zu vermischen und dabei zu lösen.

Vorteil der Eindosierung des PDNs als Schmelze oder Feststoff in den flüssigen Ammoniak und den praktisch aus Xylylendiamin und Ammoniak bestehenden Teilstrom des Reaktorumlaufs ist, dass das Phthalodinitril bereits unmittelbar nach der Vermischung verdünnt und bei einer Temperatur deutlich unter 120°C vorliegt, so dass eine ungewünschte Reaktion zwischen Nitril und Produkt weitgehend unterdrückt werden kann. Außerdem kann die Schmelzedosierung bei einem Druck erfolgen, der kleiner ist als der Reaktordruck, wodurch eine kostengünstigere Schmelzepumpe eingesetzt werden kann. Die Lösung kann dann anschließend auf den gewünschten Reaktordruck komprimiert werden. Durch Verwendung eines Teilstromes des Reaktorumlaufes kann der darin enthaltene Ammoniak ohne vorherige Aufarbeitung erneut zum Lösen von Phthalodinitril verwendet werden. Somit lässt sich das für die Reaktion optimale Verhältnis von Phthalodinitril und Ammoniak einstellen. Der größte Teil des Ammoniaks wird somit im Kreis gefahren und nur die kleine Menge an Ammoniak, welche mit dem Reaktionsprodukt zur Aufarbeitung gefahren wird (z.B. Strom [16] in Abb. 3), muss durch Druckdestillation aufgearbeitet werden. Eine zusätzliche Menge an frischem oder aufgearbeitetem Ammoniak zum Lösen des Phthalodinitrils bei niedriger Temperatur ist nicht erforderlich. Somit ist es möglich, die Lösung von Phthalodinitril bei niedriger Temperatur durchzuführen und gleichzeitig nur die Mindestmenge an Ammoniak aufzuarbeiten. Die Anforderungen an die einzusetzenden Maschinen und Apparate entsprechen dem angebotenen Standard, wodurch Apparate- und Wartungskosten klein gehalten werden können.

Das Zufahren und Lösen des Phthalodinitrils in die beiden flüssigen Ströme a und b erfordert eine Mischeinrichtung, bevorzugt eine Mischdüse oder einen Mischbehälter. Dabei ist es möglich, die Ströme aus Ammoniak (Strom a) und der Mischung von Ammoniak und Xylylendiamin (Strom b) zuerst zu mischen und dann PDN, z.B. IPDN, in dieser Mischung zu lösen. Es ist aber auch möglich, alle drei Ströme gleichzeitig und getrennt der Mischeinrichtung zuzuführen.

Eine Mischdüse kann im einfachsten Fall durch ein Rohrleitungs-T-Stück realisiert werden. Bevorzugt weist der Düsenmund eine Verjüngung auf.

Bei Einsatz einer Mischdüse werden zwei oder mehr Ströme getrennt zugeführt und im anschließenden Rohr auf Grund der herrschenden Turbulenz vermischt und homogenisiert. Vorteilhaft kann zusätzlich ein statischer Mischer nachgeschaltet werden. Es wird jedoch kein zusätzlicher Apparat, wie etwa ein Rührkessel zum Lösen von (festem oder flüssigem) Phthalodinitril in einem Lösungsmittel benötigt.

Bevorzugt ist die Mischeinrichtung am Ort der Phthalodinitrilzufuhr in die Ströme a und b auf eine Temperatur im Bereich von 1 bis 60°C, besonders im Bereich von 5 bis 40°C und insbesondere im Bereich von 7 bis 25°C oberhalb des Schmelzpunktes des eingesetzten Phthalodinitrils beheizt.

Bevorzugt erfolgt die PDN-Zufuhr praktisch bei einem Absolutdruck zwischen 15 bar und dem Reaktordruck. Der Mindestdruck ergibt sich aus der besonders bevorzugten Randbedingung, dass bei der Vermischung und der sich einstellenden Mischtemperatur keine Verdampfung eintritt, sondern die Mischung flüssig bleibt. Er ist somit von der Ausgangstemperatur und dem Mengenverhältnis der zu mischenden Komponenten abhängig. Eine Vermischung bei niedrigem Druck, z.B. im Bereich von 25 bis 40 bar, bietet den Vorteil, dass die Schmelzepumpe nicht für den deutlich höheren Reaktordruck ausgelegt werden muss. Die PDN-Lösung in Ammoniak muss in diesem Fall jedoch noch mittels einer - konstruktiv einfacheren - Hochdruckpumpe auf den Reaktordruck komprimiert werden.

Besonders bevorzugt wird mittels einer Mischdüse als Mischeinrichtung das flüssige Phthalodinitril in die Ströme a und b eingedüst.

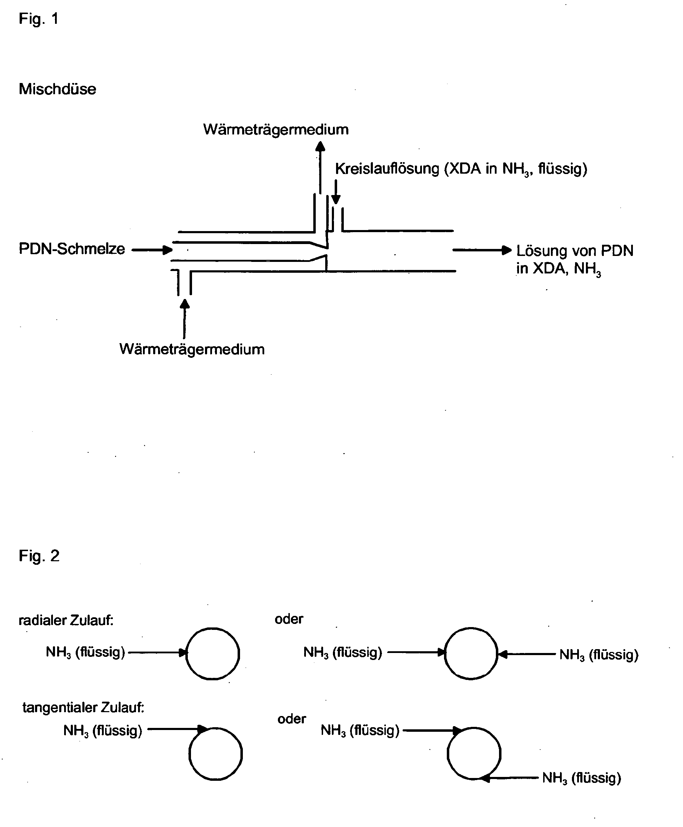

Eine bevorzugte Ausführungsform der Mischdüse ist in Abbildung 1 im Anhang dargestellt. Die Beheizung der Mischdüse kann z.B. mit Dampf, Wärmeträgeröl oder auch elektrisch erfolgen.

Der Zulauf des flüssigen Ammoniaks kann über einen oder mehrere radial oder tangential angebrachte Stutzen erfolgen, z.B. wie in der Abbildung 2 gezeigt.

Wichtig ist die lokal hohe Strömungsgeschwindigkeit (hoher Impulsstrom und Turbulenz), so dass eine schnelle Vermischung (Homogenisierung) eintritt. Bei laminarer Strömung reicht der Stoffaustausch zur Homogenisierung nicht aus und die Ströme werden nur unzureichend gemischt (Schlierenbildung).

Geeignete Mischdüsen sind z.B. in Ullmann's Encyclopedia of Industrial Chemistry, 5. Auflage, Vol. B4, Seiten 565 - 569, beschrieben.

Eine weitere besonders bevorzugte Mischeinrichtung ist ein Mischbehälter mit Umlaufkreisstrom, wie beispielsweise in Abbildung 3 gezeigt.

Die PDN-Schmelze (Strom [1], Abb. 3) wird getrennt von den anderen Strömen dem Behälter B 150 zugeführt. Flüssiger Ammoniak (Strom [2]), ein Teilstrom (Strom [13]) aus dem Umlaufkreisstrom um den Hydrierreaktor und der Umlaufstrom (Strom [4]) um den Mischbehälter können entweder getrennt oder, vereinigt und gemeinsam dem Mischbehälter B 150 zugeführt. Alternativ wird, wie in Abb. 3 gezeigt, der Ammoniak (Strom [2]) getrennt und Strom [4] und [13] vereinigt in Behälter B150 zugefahren. Die verschiedenen Zulaufströme können z.B. jeweils mit Hilfe von Düsen versprüht (verdüst) oder anderweitig zusammengeführt und vermischt werden. Zur Verbesserung der Turbulenz im Mischbehälter kann ein Teilstrom des Stromes [4] abgezweigt und über eine Mischdüse zum Behälter gefahren werden (Strom [4a]).

Dabei können für jeden Strom eine oder auch mehrere Düsen zum Einsatz kommen. Der flüssige Ammoniak kann ebenfalls versprüht werden. Falls auf den Umlaufkreisstrom (Strom [4]) um den Mischbehälter verzichtet wird, wird Ammoniak an Stelle der Lösung versprüht. Die Lösung aus dem Umlaufkreisstrom bzw. der flüssige Ammoniak werden dabei bevorzugt so versprüht, dass ein Großteil des Gasraumes erfasst wird. Insbesondere wird die Lösung bzw. der Ammoniak über die gesamte Querschnittsfläche versprüht, so dass ein Teil der Lösung bzw. ein Teil des flüssigen Ammoniaks an den Behälterwänden herunterläuft und einen Flüssigkeitsfilm bildet. Feststoffablagerungen werden so vermieden. Das PDN wird ebenfalls versprüht, jedoch bevorzugt innerhalb eines engeren Sprühkegels, der sich im wesentlichen innerhalb des Lösungs- bzw. Ammoniaksprühkegels befindet. Vorteilhaft kann das z.B. dadurch realisiert werden, dass die PDN Düse zentrisch angebracht ist und z.B. drei oder mehr Düsen für Lösung bzw. Ammoniak darum herum angeordnet sind. Die kleinen PDN Tröpfchen vermischen und lösen sich spontan im Ammoniak bzw. in der Lösung. Die Mischung wird mit der Pumpe P 150 aus dem Mischbehälter gefördert und kann in einem Wärmeübertrager W 150 auf die gewünschte Temperatur eingestellt werden. Je nach Mischungsverhältnis und Temperaturen der einzelnen Teilströme muss in W 150 geheizt oder gekühlt werden, um die gewünschte Temperatur zu erhalten. Anschließend wird die Mischung (Strom [6]) mit der Pumpe P 160 auf Reaktordruck komprimiert.

Die Mischung nach dem Wärmeübertrager W 150 wieder zum Mischbehälter zurückzufahren (Strom [4]) und mittels Düsen zu versprühen, ist aufgrund des Wärmeaustrags von Vorteil. Es kann aber auch auf den Umlaufkreisstrom verzichtet werden. Dann ist der Mengenstrom [3] gleich dem Mengenstrom [6] und der Mengenstrom [4] wird Null. Der Betriebsdruck des Behälters ist von den Verfahrensbedingungen abhängig, insbesondere von der Temperatur. Bei gegebener Zulauftemperatur des frischen Ammoniaks (Strom [2]), der PDN-Schmelze (Strom [1]) und des Teilstromes aus dem Reaktorumlauf (Strom [3]) kann durch Variation des Umlaufstromes um den Mischbehälter (Strom [4]) und Einstellung der Temperatur des Umlaufstromes nach dem Kühler W 150 die Mischtemperatur im Behälter innerhalb gewisser Grenzen, insbesondere unter Berücksichtigung der von der Temperatur abhängigen Löslichkeit von PDN in einer Mischung aus Ammoniak und Xylylendiamin gewählt werden. Damit stellt sich dann über der Lösung im Gasraum der entsprechende Dampfdruck ein. Ggf. kann im Gasraum auch noch Inertgas, z.B. Stickstoff oder Wasserstoff anwesend sein. Der Wasserstoff wird gelöst im Strom [13] eingetragen und bei der Druckentspannung auf Mischbehälterdruck freigesetzt. Aus dem Mischbehälter kann somit bei Bedarf ein Abgasstrom [17] ausgetragen werden. Vorteilhaft beträgt der Druck im Behälter B 150 nicht mehr als 40 bar, bevorzugt nicht mehr als 30 bar, besonders bevorzugt nicht mehr als 25 bar, so dass kostengünstige Apparate und Maschinen zum Einsatz kommen können. Die Hochdruckpumpe P 160 komprimiert dann die Lösung auf den Reaktordruck für die Hydrierung. Der Strom [6] wird dann mit dem restlichen Umlaufstrom um den Reaktor (Strom [12]) vermischt, z.B. mit Hilfe eines statischen Mischers A 200, wie in Abb. 3 gezeigt. Wenn der komplette Strom [11] (Strom [13] entspricht Strom [11]; Strom [12] = 0) zum Mischbehälter gefahren wird, wird, der Strom [6] direkt dem Reaktor zugefahren.

An Stelle des Mischbehälters kann auch ein Rührkessel eingesetzt werden, dessen Temperatur über einen Doppelmantel oder einen außenliegenden Umlaufkreisstrom mit Wärmeübertrager eingestellt werden kann.

In einer bevorzugten Ausführungsform des erfindungsgemäßen Verfahrens wird die resultierende flüssige Mischung aus Ammoniak, Phthalodinitril und Xylylendiamin in den Umlaufstrom um den Hydrierreaktor zugefahren, wobei der Umlaufstrom zu größer 93 Gew.-% aus flüssigem Ammoniak und Xylylendiamin besteht.

Das erfindungsgemäße Verfahren ist in einer vorteilhaften Ausführungsform weiterhin dadurch gekennzeichnet, dass das dem Hydrierreaktor bzw. der Hydrierreaktorkaskade zugefahrene Gemisch kein weiteres Lösungsmittel für Phthalodinitril enthält.

Der Phthalodinitril-Umsatz im Hydrierreaktor bzw. in der Hydrierreaktorkaskade beträgt bei einfachem Durchgang bevorzugt größer 99 %, besonders größer 99,5 %, ganz besonders größer 99,9 %, insbesondere größer 99,95 %, ganz besonders größer 99,97 %. Im Hydrierreaktor wird also durch entsprechende Einstellung der Reaktionsbedingungen (Druck, Temperatur, Molverhältnisse von PDN, NH₃ und H₂, Katalysator, Mengenströme, Verweilzeit im Reaktor) praktisch Vollumsatz gefahren.

Bevorzugt besteht der am Reaktorausgang abgezweigte flüssige Umlaufstrom um den Hydrierreaktor zu größer 94 Gew.-%, insbesondere größer 95 Gew.-%, ganz besonders größer 96 Gew.-%, weiter besonders größer 97 Gew.-%, aus flüssigem Ammoniak und Xylylendiamin; den Rest bilden Nebenkomponenten.

Nebenkomponenten im flüssigen Umlaufstrom um den Hydrierreaktor bzw. die Hydrierreaktorkaskade (Kreislaufstrom) können bei der Reaktion gebildete Nebenprodukte sowie gelöste Gase und mit dem Phthalodinitril zugefahrene Nebenkomponenten, wie z.B. Lösungsmittelreste einer vorgeschalteten PDN-Quench-Stufe (z.B. Tolunitril, Benzonitril), jedoch bevorzugt kein weiteres Lösungsmittel, z.B. organisches Lösungsmittel, für Phthalodinitril sein.

Der am Reaktorausgang abgezweigte Umlaufstrom um den Hydrierreaktor bzw. die Hydrierreaktorkaskade enthält bevorzugt im Bereich von 25 bis 90 Gew.-%, besonders 30 bis 70 Gew.-%, insbesondere 45 bis 60 Gew.-%, flüssigen Ammoniak.

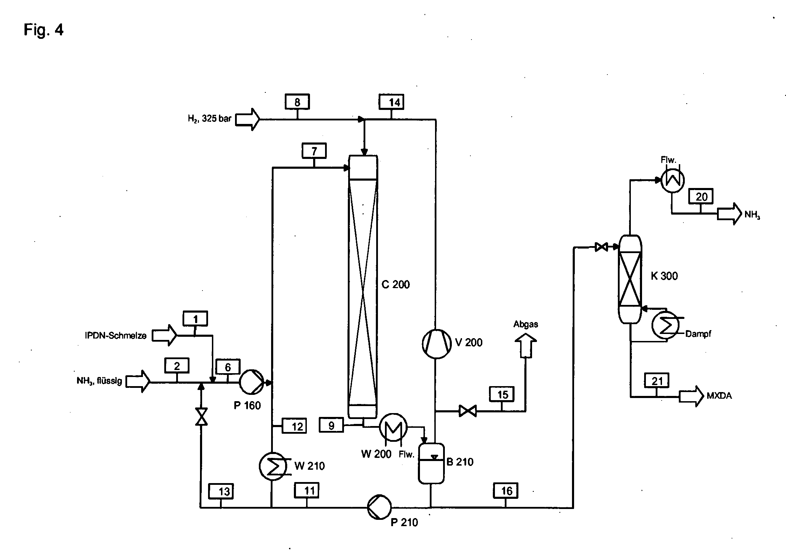

Der Teil des flüssigen Reaktoraustrags, der als Umlaufstrom direkt oder indirekt, bevorzugt kontinuierlich, zum Reaktoreingang zurückgeführt wird (siehe z.B. Strom [11] in den Abb. 3 und 4; oder auch z.B. die Summe der Ströme 12 und 13 in den Abb. 3 und 4), macht bevorzugt 20 bis 95 Gew.-%, besonders 50 bis 92 Gew.-%, insbesondere 75 bis 90 Gew.-%, des gesamten flüssigen Reaktoraustrags aus.

Das Gewichtsverhältnis von frischem Phthalodinitril-Zulaufstrom + Ammoniak-Zulaufstrom (z.B. Summe der Ströme [1] und [2] in Abb. 3 und 4) zu Umlaufstrom um den Hydrierreaktor (z.B. Strom [11] in Abb. 3 und 4) liegt bevorzugt im Bereich von 0,05 bis 5, besonders im Bereich von 0,1 bis 2,0, insbesondere im Bereich von 0,15 bis 1,0.

Die Reaktionstemperatur liegt bevorzugt im Bereich von 40 bis 150°C, besonders bevorzugt 50 bis 135°C, insbesondere 60 bis 130°C.

Die Ammoniakmenge, die Menge des Kreislaufstromes [z.B. Strom 11] und die Reaktorzulauftemperatur werden so eingestellt, dass die Reaktoraustrittstemperatur den gewünschten Maximalwert (z.B. 130°C) nicht überschreitet, da mit zunehmender Temperatur verstärkt Nebenprodukte gebildet werden. Die Reaktorzulauftemperatur wird so eingestellt (z.B. durch einen zusätzlichen Wärmeübertrager oder, bevorzugt, durch geeignete Einstellung der Temperatur der zu mischenden Ströme), dass die Reaktion hinreichend schnell verläuft und Vollumsatz erreicht wird. Wenn zunächst nur Teilumsatz erreicht wird, wird die Zulauftemperatur zum Reaktor so erhöht, dass Vollumsatz erreicht wird. Durch Variation von Kreislaufmengenstrom um den Hydrierreaktor bzw. die Hydrierreaktorkaskade und Frischmengenstrom an Ammoniak ist es somit möglich, sowohl die Eintritts- als auch, innerhalb gewisser Grenzen, die Austrittstemperatur des Reaktors einzustellen und optimal an die ablaufenden Reaktionen anzupassen und so die XDA-Ausbeute zu optimieren.

Die Hydrierung wird bevorzugt bei einem Absolutdruck im Bereich von 100 bis 300 bar, insbesondere 120 bis 220 bar, ganz besonders 150 bis 200 bar, durchgeführt.

Für die Hydrierung können dem Fachmann bekannte Katalysatoren und Reaktoren (insbesondere Rohrreaktoren oder Rohrbündelreaktoren; Festbett- oder Suspensionsfahrweise) angewendet werden.

Bei der Katalysatorfestbettfahrweise ist sowohl die Sumpf- als auch die Rieselfahrweise möglich. Bevorzugt ist eine Rieselfahrweise.

Bevorzugt wird der Reaktor bzw. die Reaktoren adiabat betrieben, während die entstehende Reaktionswärme über einen im Umlaufkreisstrom eingebauten Kühler sowie optional mit dem verwendeten Kreisgas abgeführt wird. Dies erhöht zusätzlich die Selektivität der Reaktion durch die weitere Unterdrückung von Nebenprodukten. Alternativ ist aber auch ein gekühlter Reaktor bzw. Reaktoren, beispielsweise ein Rohrbündelreaktor, einsetzbar.

Bevorzugt sind Katalysatoren, die Kobalt und/oder Nickel und/oder Eisen, als Vollkatalysator oder auf einem inerten Träger, enthalten. Anwendbar ist hier auch eine Kombination von verschiedenen Katalysatoren.

Besonders bevorzugt wird die Hydrierung an einem Mangan-dotierten Cobalt-Vollkatalysator durchgeführt.

Geeignete Katalysatoren sind beispielsweise Raney-Nickel, Raney-Cobalt, Co-Voll-kontakt, Titan-dotiertes Cobalt auf Träger (JP-A-2002 205980), Ni auf SiO₂-Träger (WO-A-2000/046179), Co/Ti/Pd auf SiO₂-Träger (CN-A-1 285 343, CN-A-1 285 236) und Nickel und/oder Cobalt auf Zirkoniumdioxid-Träger (EP-A1-1 262 232).

Beispiele für weitere geeignete Katalysatoren finden sich z.B. in den Anmeldungen GB-A-852,972 (Äquivalent: DE-A-11 19 285) (BASF AG), DE-A-12 59 899 (BASF AG) und den US Patenten Nr. 3,069,469 (California Research Corp.) und 4,482,741 (UOP Inc.).

Besonders bevorzugte Katalysatoren sind die in EP-A1-742 045 (BASF AG) offenbarten Cobalt-Vollkontakte, dotiert mit Mn, P, und Alkalimetall (Li, Na, K, Rb, Cs).

Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A-963 975 (BASF AG) offenbarten Katalysatoren, deren katalytisch aktive Masse vor der Behandlung mit Wasserstoff
22 bis 40 Gew.-% ZrO₂
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, wobei das molare Ni: :Cu-Verhältnis größer 1 ist,
15 bis 50 Gew.-% sauerstoffhaltige Verbindungen des Kobalts, berechnet als CoO,
0 bis 10 Gew.-% sauerstoffhaltige Verbindungen des Aluminiums und/oder Mangans, berechnet als Al₂O₃ bzw. MnO₂,
und keine sauerstoffhaltigen Verbindungen des Molybdäns enthält,
beispielsweise der in loc. cit., Seite 17, offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO,
die
in EP-A-696 572 (BASF AG) offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂, enthält, beispielsweise der in loc. cit., Seite 8, offenbarte Katalysator mit der Zusammensetzung 31,5 Gew.-% ZrO₂ 50 Gew.-% NiO, 17 Gew.-% CuO und 1,5 Gew.-% MoOs,
und die in WO-A-99/44984 (BASF AG) beschriebenen Katalysatoren enthaltend (a) Eisen oder eine Verbindung auf der Basis von Eisen oder deren Gemische, (b) von
0,001 bis 0,3 Gew.-% bezogen auf (a) eines Promoters auf der Basis von 2, 3,4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Das erfindungsgemäße Verfahren lässt sich z.B. wie folgt ausführen:
In Abbildung 4 ist eine mögliche Anordnung des Hydrierreaktors einschließlich des optionalen Umlaufkreisstromes [12] und eines Wärmeübertragers (W 210) dargestellt.

Die Phthalodinitrilschmelze wird als Strom [1] zugeführt, mit dem flüssigen Ammoniak [2] sowie dem Teilstrom aus dem Umlaufkreisstrom um den Reaktor [13] gemischt. Die Mischung wird entweder mit dem optional vorhandenen Umlaufkreisstrom [12] gemischt oder direkt dem Reaktor zugefahren. Zum besseren Vermischen mit dem Umlaufkreisstrom kann z.B. ein statischer Mischer verwendet werden.

Wasserstoff und ggf. Kreisgas können mittels eines optionalen Wärmeübertragers auf die gewünschte Reaktorzulauftemperatur erwärmt werden. Gas und Flüssigkeit können gemeinsam oder - bevorzugt - getrennt dem Reaktor zugefahren werden. Bevorzugt wird die Temperatur der zu mischenden Ströme mittels Wärmeübertragern so eingestellt, dass hinter dem Mischpunkt kein Wärmeübertrager mehr erforderlich ist. Im Reaktor erfolgt die Hydrierung praktisch quantitativ, so dass im Reaktionsaustrag praktisch kein Phthalodinitril mehr vorhanden ist. Der Reaktionsaustrag kann dann abgekühlt werden, und Gas und Flüssigkeit werden unter Druck in einem Hochdruckabscheider getrennt. Bei Kreisflüssigkeitsfahrweise wird ein Teil der Flüssigkeit aus dem Reaktionsaustrag ohne Aufarbeitung im Kreis gefahren (Strom [11]). Ansonsten wird der Reaktionsaustrag teilweise dem Mischbehälter (Strom [13] und teilweise der Aufarbeitung (Strom [16]) zugeführt. Ein Teil des Gases kann ausgeschleust werden (Strom [15]), um die Aufpegelung von Inerten (CO, N₂, CH₄, Edelgase, etc.) zu vermeiden. Der größte Teil des Gases wird über einen Verdichter zum Reaktoreingang zurückgeführt. Bei nicht zu hohem Druckverlust im Reaktor kann hierzu bevorzugt auch eine Ejektorstrahldüse ("Wasserstrahlpumpe") verwendet werden. Insgesamt kann die Kreisgasmenge in weiten Bereichen variiert werden, etwa vom mehrfachen der Frischgasmenge bis hin zu Null (Fahrweise ohne Kreisgas). Die Kreisgasfahrweise ist günstig, um den Reaktor für einen guten Stoffübergang ausreichend gasseitig zu belasten und um für Inertgase einen hinreichenden Schleppstrom zur Verfügung zu stellen, um sie am Reaktorausgang ausschleusen zu können. Zusätzlich kann ein Teil der Reaktionswärme mit dem Gasstrom abgeführt werden. Mit zunehmender Temperatur verdampft eine zunehmende Menge an Ammoniak, was den kühlenden Effekt des Kreisgases noch verstärkt. Der Reaktionsaustrag (Strom [16]) wird dann zunächst einer Druckdestillation zugeführt, in der flüssiger Ammoniak über Kopf (Strom [20]) und weitgehend ammoniakfreies, rohes Xylylendiamin über Sumpf (Strom [21]) gewonnen werden, wobei der Ammoniak in kondensierter Form wieder der Hydrierstufe (als Strom [2]) zugeführt werden kann. Das rohe Xylylendiamin wird z.B. durch Destillation weiter gereinigt.

Im erfindungsgemäßen Verfahren kann das Gewichtsverhältnis der Frischzuläufe von Dinitril und Ammoniak (z.B. gemäß Abbildung 3 oder 4 das Verhältnis von Strom [1] zu Strom [2]) um so größer gewählt werden, je größer der aus dem Strom [11] abgezweigten Teilstrom [13] zum Mischbehälter ist. Der Strom [13] ist je nach Menge des Stromes [2] nach unten hin durch die Löslichkeit von Phthalodinitril in der Mischung aus flüssigem Ammoniak und Phthalodinitril bei der gegebenen Temperatur begrenzt (z.B. beträgt die Löslichkeit von IPDN in NH₃ bei 60°C ca. 44 Gew.-% und in meta-Xylylendiamin ca. 15 Gew.-%. In einer Mischung aus etwa gleichen Massenanteilen von meta-Xylylendiamin und flüssigem Ammoniak beträgt die Löslichkeit ca. 30 Gew.-%).

Bevorzugt beträgt das Einsatzstoff-Gewichtsverhältnis von Dinitril (Strom [1]) zu Ammoniak (Strom [2]) 1 : 0,5 bis 1 : 2, vorzugsweise 1 : 0,7 bis 1 : 1,5, besonders bevorzugt 1 : 0,9 bis 1 : 1,2.

Das Gewichtsverhältnis des rückgeführten Teilstromes [z.B. Strom 13] aus dem Umlaufstrom zum Frischzulauf (Summe aus frischem Phthalodinitril- und Ammoniak-Zulaufstrom, z.B. der Ströme [1] und [2]) beträgt bevorzugt 1 : 0,3 bis 1 : 2, vorzugsweise 1 : 0,5 bis 1 : 1,5 , besonders bevorzugt 1 : 0,7 bis 1 : 1,1.

Bei der Fahrweise ohne direkte Kreisflüssigkeit (Strom [12] = 0) um den Hydrierreaktor bzw. Hydrierreaktoren, d.h. bei dem Strom, der zumindest als Teilstrom aus dem Umlaufstrom um den Hydrierreaktor abgezogen wird (Strom b), handelt es sich um den gesamten Umlaufkreisstrom, beträgt das Einsatzstoff-Gewichtsverhältnis von Dinitril zu Ammoniak bevorzugt 1 : 0,5 bis 1 : 2, vorzugsweise 1 : 0,7 bis 1 : 1,5, besonders bevorzugt 1 : 0,9 bis 1 : 1,2, d.h. es ist bevorzugt das gleiche wie im Fall mit direktem Kreislauf um den Hydrierreaktor wie oben beschrieben.

Das Gewichtsverhältnis des zurückgeführten Teilstromes [z.B. Strom13] aus dem Reaktoraustrag zum Frischzulauf (Summe aus frischem Phthalodinitril- und Ammoniak-Zulaufstrom, z.B. Summe der Ströme [1] und [2]) beträgt in diesem Fall 2 : 1 bis 10 : 1, vorzugsweise 3 : 1 bis 8 : 1 , besonders bevorzugt 4,5 : 1 bis 6 : 1.

### Isolierung des XDAs:

Nach der Hydrierung wird der eingesetzte Ammoniak abgetrennt, z.B. abdestilliert.

Bevorzugt erfolgt eine Reinigung des Xylylendiamins durch Abdestillation leichtersiedender Nebenprodukte (bei gleichem Druck) über Kopf und destillativer Abtrennung von schwerersiedenden Verunreinigungen über Sumpf.

Besonders bevorzugt ist die Fahrweise, in der man nach der Hydrierung den Ammoniak sowie gegebenenfalls leichtsiedende Nebenprodukte über Kopf abdestilliert und danach schwerersiedende Verunreinigungen vom Xylylendiamin destillativ über Sumpf abtrennt.

In einer besonderen Ausführungsform kann die Abtrennung leichter- und schwerersiedender Nebenprodukte auch in einer Seitenabzugs- oder Trennwandkolonne erfolgen, wobei reines Xylylendiamin über einen flüssigen oder gasförmigen Seitenabzug gewonnen wird.

Je nach gewünschter Reinheit wird das Produkt (XDA) zusätzlich mit einem organischen Lösungsmittel, bevorzugt einem aliphatischen Kohlenwasserstoff, insbesondere einem cycloaliphatischen Kohlenwasserstoff, ganz besonders Cyclohexan oder Methylcyclohexan, extrahiert.

Diese Reinigung durch Extraktion kann z.B. gemäß DE-A-1 074 592 (BASF AG) erfolgen.

### Beispiele

In den Beispielen ist die Mischung der Ströme berechnet (durch Addition der Einzelstrommengen), die Hydrierung der resultierenden Mischung wurde experimentell durchgeführt, wobei Kreisläufe nicht geschlossen waren.

### Beispiel 1:

90 g/h geschmolzenes IPDN (kommerzielles, geschupptes IPDN, welches durch Erhitzen auf ca. 170°C aufgeschmolzen wurde) werden in einem Behälter bei 60°C mit 178 g/h aus dem Kreislaufstrom [13] und 94 g/h Frisch-Ammoniak [2] vermischt und dabei gelöst. Es entsteht eine Lösung mit 25 Gew.% IPDN. Bei 60°C liegt die Löslichkeit von IPDN in der Mischung bei etwa 30 Gew.%. Die Mischtemperatur kann durch die Temperatur und den Mengenstrom des Kreislaufstromes [4] eingestellt werden.

Wenn Ammoniak bei 30°C, der Kreislaufstrom [13] bei 50°C und IPDN bei 170°C zugefahren werden, kann der Strom [4] z.B. 470 g/h bei 58°C betragen, um zur Mischtemperatur von 60°C zu kommen, wobei Wärmeverluste nicht berücksichtigt sind. Der Siededruck im Mischbehälter beträgt dann 23,3 bar (abs.), d.h. oberhalb dieses Druckes bleibt die Mischung flüssig und es findet keine Verdampfung von Ammoniak statt. Die. Lösung wird in einen Kreislaufstrom (ca. 839 g/h) bestehend aus dem flüssigen Rückführstrom des Reaktoraustrags gefahren. Die IPDN-Konzentration am Reaktoreintritt beträgt somit 7,5 Gew.%. Pro mol IPDN liegen 50 mol NH₃ am Reaktoreintritt vor.

Die erhaltene Reaktionsmischung wurde kontinuierlich in einen Rohreaktor an einem Kobalt-Vollkontakt bei 90°C und 200 bar hydriert. Der abgezogene Teil des Reaktoraustrags wurde in einer Ammoniakkolonne vom Großteil der Ammoniakmenge befreit und über GC-untersucht. Bei Vollumsatz des eingesetzten IPDN (d.h. Umsatz größer 99,95 %; per GC kein Edukt mehr nachweisbar) lag die Selektivität bei 92 %.

In anschließenden Destillationsschritten wurden zuerst Rest-Ammoniak und leichtsiedende Nebenkomponenten abgetrennt. Nach Abtrennung der hochsiedenden Verunreinigungen über Sumpf wurde MXDA als Kopfprodukt einer Destillationskolonne in einer Reinheit von mehr als 99,9 Gew.-% erhalten.

### Beispiel 2:

90 g/h geschmolzenes IPDN (kommerzielles, geschupptes IPDN, welches durch Erhitzen auf ca. 170°C aufgeschmolzen wurde) werden in einem Behälter bei 60°C mit 320 g/h aus dem Kreislaufstrom [13] und 40 g/h Frisch-Ammoniak [2] vermischt und dabei gelöst. Es entsteht eine Lösung mit 20 Gew.% IPDN. Bei 60°C liegt die Löslichkeit von IPDN in der Mischung bei etwa 25 Gew.%. Die Mischtemperatur kann durch die Temperatur und den Mengenstrom des Kreislaufstromes [4] eingestellt werden.

Wenn Ammoniak bei 30°C, der Kreislaufstrom [13] bei 45°C und IPDN bei 170°C zugefahren werden, kann der Strom [4] z.B. 527 g/h bei 58°C betragen, um zur Mischtemperatur von 60°C zu kommen, wobei Wärmeverluste nicht berücksichtigt sind. Der Siededruck im Mischbehälter beträgt dann 20,2 bar (abs.), d.h. oberhalb dieses Druckes bleibt die Mischung flüssig und es findet keine Verdampfung von Ammoniak statt. Die Lösung wird in einen Kreislaufstrom (ca. 750 g/h) bestehend aus dem flüssigen Rückführstrom des Reaktoraustrags gefahren. Die IPDN-Konzentration am Reaktoreintritt beträgt somit 7,5 Gew.%. Pro mol IPDN liegen 30 mol NH₃ am Reaktoreintritt vor.

Die erhaltene Reaktionsmischung wurde kontinuierlich in einen Rohreaktor an einem Kobalt-Vollkontakt bei 90°C und 200 bar hydriert. Der abgezogene Teil des Reaktoraustrags wurde in einer Ammoniakkolonne vom Großteil der Ammoniakmenge befreit und über GC-untersucht. Bei Vollumsatz des eingesetzten IPDN (d.h. Umsatz größer 99,95 %; per GC kein Edukt mehr nachweisbar) lag die Selektivität bei 88 %.

In anschließenden Destillationsschritten wurden zuerst Rest-Ammoniak und leichtsiedende Nebenkomponenten abgetrennt. Nach Abtrennung der hochsiedenden Verunreinigungen über Sumpf wurde MXDA als Kopfprodukt einer Destillationskolonne in einer Reinheit von mehr als 99,9 Gew.-% erhalten.

## Patentansprüche

1. Verfahren zur Herstellung von Xylylendiamin durch kontinuierliche Hydrierung von Phthalodinitril an einem Heterogenkatalysator in Gegenwart von flüssigem Ammoniak in einem Reaktor, wobei ein Teil des Reaktoraustrags als flüssiger Umlaufstrom kontinuierlich zum Reaktoreingang zurückgeführt wird (Umlaufkreisfahrweise), **dadurch gekennzeichnet, dass** mittels einer Mischeinrichtung Phthalodinitril als Schmelze oder in fester Form mit
einem Strom von flüssigem Ammoniak (Strom a) und
einem weiteren Strom, der zumindest als Teilstrom aus dem Umlaufstrom um den Hydrierreaktor abgezogen wird (Strom b),
oder einer Mischung aus den Strömen a und b gemischt wird und die resultierende flüssige Mischung in den Hydrierreaktor gefahren wird, sodass der Umlaufstrom mindestens teilweise nicht direkt zum Reaktor zurückgeführt wird, sondern indirekt über die Mischeinrichtung.

2. Verfahren nach Anspruch 1 zur Herstellung von meta-Xylylendiamin durch Hydrierung von Isophthalodinitril.

3. Verfahren nach den Ansprüchen 1 oder 2, **dadurch gekennzeichnet, dass** die Mischeinrichtung am Ort der Phthalodinitrilzufuhr in die Ströme a und b auf eine Temperatur im Bereich von 1 bis 60°C oberhalb des Schmelzpunktes des eingesetzten Phthalodinitrils beheizt ist.

4. Verfahren nach einem der vorhergehenden Ansprüche, **dadurch gekennzeichnet, dass** mittels einer Mischdüse als Mischeinrichtung flüssiges Phthalodinitril in die Ströme a und b eingedüst wird.

5. Verfahren nach einem der Ansprüche 1 bis 3, **dadurch gekennzeichnet, dass** in einem Mischbehälter als Mischeinrichtung flüssiges Phthalodinitril und flüssiger Ammoniak (Strom a) und der Strom, der zumindest als Teilstrom aus dem Umlaufstrom um den Hydrierreaktor abgezogen wird (Strom b), oder eine Mischung aus den Strömen a und b eingedüst werden.

6. Verfahren nach den Ansprüchen 1 oder 2, **dadurch gekennzeichnet, dass** festes Phthalodinitril in eine Mischeinrichtung eingebracht wird, zu der flüssiger Ammoniak (Strom a) und der Strom, der zumindest als Teilstrom aus dem Umlaufstrom um den Hydrierreaktor abgezogen wird (Strom b), oder eine Mischung aus den Strömen a und b zugefahren wird.

7. Verfahren nach einem der vorhergehenden Ansprüche, **dadurch gekennzeichnet, dass** die resultierende flüssige Mischung aus Ammoniak, Phthalodinitril und Xylylendiamin in den Umlaufstrom um den Hydrierreaktor zugefahren wird, wobei der Umlaufstrom zu größer 93 Gew.-% aus flüssigem Ammoniak und Xylylendiamin besteht.

8. Verfahren nach einem der vorhergehenden Ansprüche, **dadurch gekennzeichnet, dass** das dem Hydrierreaktor zugefahrene Gemisch kein weiteres Lösungsmittel für Phthalodinitril enthält.

9. Verfahren nach einem der vorhergehenden Ansprüche, **dadurch gekennzeichnet, dass** der Phthalodinitril-Umsatz im Hydrierreaktor bei einfachem Durchgang größer 99 % beträgt.

10. Verfahren nach einem der Ansprüche 1 bis 8, **dadurch gekennzeichnet, dass** der Phthalodinitril-Umsatz im Hydrierreaktor bei einfachem Durchgang größer 99,5 % beträgt.

11. Verfahren nach einem der vorhergehenden Ansprüche, **dadurch gekennzeichnet, dass** der Umlaufstrom um den Hydrierreaktor zu größer 94 Gew.-% aus flüssigem Ammoniak und Xylylendiamin besteht.

12. Verfahren nach einem der vorhergehenden Ansprüche, **dadurch gekennzeichnet, dass** der Umlaufstrom um den Hydrierreaktor im Bereich von 25 bis 90 Gew.-% flüssigen Ammoniak enthält.

13. Verfahren nach einem der vorhergehenden Ansprüche, **dadurch gekennzeichnet, dass** der Teil des flüssigen Reaktoraustrags, der als Umlaufstrom direkt oder indirekt zum Reaktoreingang zurückgeführt wird, 20 bis 95 Gew.-% des gesamten flüssigen Reaktoraustrags ausmacht.

14. Verfahren nach einem der vorhergehenden Ansprüche, **dadurch gekennzeichnet, dass** das Gewichtsverhältnis von frischem Phthalodinitril-Zulaufstrom + Ammoniak-Zulaufstrom zu Umlaufstrom um den Hydrierreaktor im Bereich von 0,05 bis 5 liegt.

15. Verfahren nach einem der vorhergehenden Ansprüche, **dadurch gekennzeichnet, dass** das Gewichtsverhältnis des zurückgeführtem Teilstroms aus dem Umlaufstrom um den Hydrierreaktor zur Summe aus frischem Phthalodinitril-Zulaufstrom und Ammoniak-Zulaufstrom im Bereich von 1 : 0,3 bis 1 : 2 liegt.

16. Verfahren nach einem der Ansprüche 1 bis 6 oder 8 bis 15, **dadurch gekennzeichnet, dass** es sich bei dem Strom, der zumindest als Teilstrom aus dem Umlaufstrom um den Hydrierreaktor abgezogen wird (Strom b), um den gesamten Umlaufkreisstrom handelt und das Gewichtsverhältnis des zurückgeführten Teilstromes aus dem Reaktoraustrag zur Summe aus frischem Phthalodinitril-Zulaufstrom und Ammoniak-Zulaufstrom im Bereich von 2 : 1 bis 10 : 1 liegt.

17. Verfahren nach einem der vorhergehenden Ansprüche, **dadurch gekennzeichnet, dass** die Hydrierung bei einer Temperatur im Bereich von 40 bis 150°C durchgeführt wird.

18. Verfahren nach einem der vorhergehenden Ansprüche, **dadurch gekennzeichnet, dass** die Hydrierung bei einem Absolutdruck im Bereich von 100 bis 300 bar durchgeführt wird.

19. Verfahren nach einem der vorhergehenden Ansprüche, **dadurch gekennzeichnet, dass** die Hydrierung an einem Katalysator enthaltend Ni, Co und/oder Fe, als Vollkatalysator oder auf einem inerten Träger, durchgeführt wird.

20. Verfahren nach einem der vorhergehenden Ansprüche, **dadurch gekennzeichnet, dass** die Hydrierung an einem Mangan-dotierten Cobalt-Vollkatalysator durchgeführt wird.

21. Verfahren nach einem der vorhergehenden Ansprüche, **dadurch gekennzeichnet, dass** der Katalysator in einem Rohrreaktor oder Rohrbündelreaktor als Festbett angeordnet ist.

22. Verfahren nach dem vorhergehenden Anspruch, **dadurch gekennzeichnet, dass** der Hydrierreaktor in Rieselfahrweise betrieben wird.

23. Verfahren nach einem der vorhergehenden Ansprüche, **dadurch gekennzeichnet, dass** der Hydrierreaktor adiabat betrieben wird.

24. Verfahren nach einem der vorhergehenden Ansprüche, **dadurch gekennzeichnet, dass** dem Umlaufstrom um den Hydrierreaktor in einem Kühler Wärme entzogen wird.

25. Verfahren nach einem der vorhergehenden Ansprüche, **dadurch gekennzeichnet, dass** als Hydrierreaktor eine Hydrierreaktorkaskade eingesetzt wird.

26. Verfahren nach einem der vorhergehenden Ansprüche, **dadurch gekennzeichnet, dass** nach der Hydrierung eine Reinigung des Xylylendiamins durch Abdestillation des Ammoniaks sowie gegebenenfalls leichtersiedender Nebenprodukte über Kopf und destillativer Abtrennung von schwerersiedenden Verunreinigungen über Sumpf erfolgt.

27. Verfahren nach dem vorhergehenden Anspruch, **dadurch gekennzeichnet, dass** das Xylylendiamin nach der Destillation zur weiteren Reinigung mit einem organischem Lösungsmittel extrahiert wird.

28. Verfahren nach dem vorhergehenden Anspruch, **dadurch gekennzeichnet, dass** man zur Extraktion Cyclohexan oder Methylcyclohexan verwendet.

## Claims

1. A process for preparing xylylenediamine by continuously hydrogenating phthalonitrile over a heterogeneous catalyst in the presence of liquid ammonia in a reactor, a portion of the reactor effluent being recycled continuously to the reactor inlet as a liquid circulation stream (circulation mode), which comprises, by means of a mixing unit, drawing off phthalonitrile as a melt or in solid form with
a stream of liquid ammonia (stream a) and
drawing off a further stream which is drawn off at least partly as a substream from the circulation stream around the hydrogenation reactor (stream b), or mixing a mixture of streams a and b and conducting the resulting liquid mixture into the hydrogenation reactor, in such a way that the circulation stream is at least partly not recycled directly to the reactor, but rather indirectly via the mixing unit.

2. The process according to claim 1 for preparing meta-xylylenediamine by hydrogenating isophthalonitrile.

3. The process according to claims 1 and 2, wherein the mixing unit, at the location of the phthalonitrile feed into streams a and b, is heated to a temperature in the range from 1 to 60°C above the melting point of the phthalonitrile used.

4. The process according to any of the preceding claims, wherein liquid phthalonitrile is sprayed into streams a and b by means of a mixing nozzle as the mixing unit.

5. The process according to any of claims 1 to 3, wherein, in a mixing vessel as the mixing unit, liquid phthalonitrile and liquid ammonia (stream a) and the stream which is drawn off at least partly as a substream from the circulation stream around the hydrogenation reactor (stream b), or a mixture of streams a and b are sprayed in.

6. The process according to claims 1 and 2, wherein the solid phthalonitrile is introduced into a mixing unit, to which is fed liquid ammonia (stream a) and the stream which is drawn off at least partly as a substream from the circulation stream around the hydrogenation reactor (stream b), or a mixture of streams a and b.

7. The process according to any of the preceding claims, wherein the resulting liquid mixture of ammonia, phthalonitrile and xylylenediamine is conducted into the circulation stream around the hydrogenation reactor, the circulation stream consisting to an extent of greater than 93% by weight of liquid ammonia and xylylenediamine.

8. The process according to any of the preceding claims, wherein the mixture conducted to the hydrogenation reactor does not comprise any further solvent for phthalonitrile.

9. The process according to any of the preceding claims, wherein the phthalonitrile conversion in the hydrogenation reactor in single pass is greater than 99%.

10. The process according to any of claims 1 to 8, wherein the phthalonitrile conversion in the hydrogenation reactor in single pass is greater than 99.5%.

11. The process according to any of the preceding claims, wherein the circulation stream around the hydrogenation reactor consists to an extent of greater than 94% by weight of liquid ammonia and xylylenediamine.

12. The process according to any of the preceding claims, wherein the circulation stream around the hydrogenation reactor comprises in the range from 25 to 90% by weight of liquid ammonia.

13. The process according to any of the preceding claims, wherein the portion of the liquid reactor effluent which is recycled directly or indirectly to the reactor inlet as the circulation stream makes up from 20 to 95% by weight of the overall liquid reactor effluent.

14. The process according to any of the preceding claims, wherein the weight ratio of fresh phthalonitrile feed stream + ammonia feed stream to circulation stream around the hydrogenation reactor is in the range from 0.05 to 5.

15. The process according to any of the preceding claims, wherein the weight ratio of recycled substream from the circulation stream around the hydrogenation reactor to the sum of fresh phthalonitrile feed stream and ammonia feed stream is in the range from 1 : 0.3 to 1 : 2.

16. The process according to any of claims 1 to 6 or 8 to 15, wherein the stream which is drawn off at least partly as a substream from the circulation stream around the hydrogenation reactor (stream b) is the entire circulation stream and the weight ratio of the recycled substream from the reactor effluent to the sum of fresh phthalonitrile feed stream and ammonia feed stream is in the range from 2 : 1 to 10 : 1.

17. The process according to any of the preceding claims, wherein the hydrogenation is carried out at a temperature in the range from 40 to 150°C.

18. The process according to any of the preceding claims, wherein the hydrogenation is carried out at an absolute pressure in the range from 100 to 300 bar.

19. The process according to any of the preceding claims, wherein the hydrogenation is carried out over a catalyst comprising Ni, Co and/or Fe as an unsupported catalyst or on an inert support.

20. The process according to any of the preceding claims, wherein the hydrogenation is carried out over a manganese-doped unsupported cobalt catalyst.

21. The process according to any of the preceding claims, wherein the catalyst is arranged as a fixed bed in a tubular reactor or tube bundle reactor.

22. The process according to the preceding claim, wherein the hydrogenation reactor is operated in trickle mode.

23. The process according to any of the preceding claims, wherein the hydrogenation reactor is operated adiabatically.

24. The process according to any of the preceding claims, wherein heat is removed in a cooler from the circulation stream around the hydrogenation reactor.

25. The process according to any of the preceding claims, wherein the hydrogenation reactor used is a hydrogenation reactor battery.

26. The process according to any of the preceding claims, wherein, after the hydrogenation, the xylylenediamine is purified by distilling off the ammonia and any lower-boiling by-products overhead and distillatively removing higher-boiling impurities via the bottom.

27. The process according to the preceding claim, wherein the xylylenediamine, after the distillation, is further purified by extracting with an organic solvent.

28. The process according to the preceding claim, wherein cyclohexane or methylcyclohexane is used for the extraction.

## Revendications

1. Procédé pour la préparation de xylylènediamine par hydrogénation continue de phtalodinitrile sur un catalyseur hétérogène en présence d'ammoniac liquide dans un réacteur, une partie du produit évacué du réacteur étant recyclée comme flux en circulation liquide en boucle en continu vers l'entrée du réacteur (conduite par circulation en boucle), **caractérisé en ce qu'**au moyen d'un dispositif de mélange, le phtalodinitrile est mélangé, sous forme de masse fondue ou sous forme solide avec
- un flux d'ammoniac liquide (flux a) et
- un autre flux qui est soutiré au moins en tant que flux partiel du flux en boucle autour du réacteur d'hydrogénation (flux b), ou
- un mélange des flux a et b et le mélange liquide résultant est guidé dans le réacteur d'hydrogénation, de manière telle que le flux en boucle n'est, du moins en partie, pas recyclé directement dans le réacteur, mais indirectement via le dispositif de mélange.

2. Procédé selon la revendication 1 pour la préparation de méta-xylylènediamine par hydrogénation d'isophtalodinitrile.

3. Procédé selon les revendications 1 ou 2, **caractérisé en ce que** le dispositif de mélange, au niveau de l'alimentation en phtalodinitrile dans les flux a et b est chauffé à une température dans la plage de 1 à 60°C au-dessus du point de fusion du phtalodinitrile utilisé.

4. Procédé selon l'une quelconque des revendications précédentes, **caractérisé en ce que** phtalodinitrile liquide est injecté au moyen un pulvérisateur de mélange comme dispositif de mélange dans les flux a et b.

5. Procédé selon l'une quelconque des revendications 1 à 3, **caractérisé en ce qu'**on injecte dans un récipient de mélange, comme dispositif de mélange, du phtalodinitrile liquide et de l'ammoniac liquide (flux a) et le flux qui est soutiré au moins en tant que flux partiel du flux en boucle autour du réacteur d'hydrogénation (flux b), ou un mélange des flux a et b.

6. Procédé selon les revendications 1 ou 2, **caractérisé en ce que** du phtalodinitrile solide est introduit dans un dispositif de mélange, dans lequel on introduit de l'ammoniac liquide (flux a) et le flux qui est soutiré, au moins en tant que flux partiel, du flux en boucle autour du réacteur d'hydrogénation (flux b), ou un mélange des flux a et b.

7. Procédé selon l'une quelconque des revendications précédentes, **caractérisé en ce que** le mélange liquide résultant d'ammoniac, de phtalodinitrile et de xylylènediamine est introduit dans le flux en boucle autour du réacteur d'hydrogénation, le flux en boucle étant constitué, à raison de plus de 93% en poids, d'ammoniac et de xylylènediamine.

8. Procédé selon l'une quelconque des revendications précédentes, **caractérisé en ce que** le mélange introduit dans le réacteur d'hydrogénation ne contient pas d'autre solvant pour le phtalodinitrile.

9. Procédé selon l'une quelconque des revendications précédentes, **caractérisé en ce que** la conversion de phtalodinitrile dans le réacteur d'hydrogénation est supérieure à 99% en un seul passage.

10. Procédé selon l'une quelconque des revendications 1 à 8, **caractérisé en ce que** la conversion de phtalodinitrile dans le réacteur d'hydrogénation est supérieure à 99,5% en un seul passage.

11. Procédé selon l'une quelconque des revendications précédentes, **caractérisé en ce que** le flux en boucle autour du réacteur d'hydrogénation est constitué, à raison de plus de 94% en poids, d'ammoniac liquide et de xylylènediamine.

12. Procédé selon l'une quelconque des revendications précédentes, **caractérisé en ce que** le flux en boucle autour du réacteur d'hydrogénation contient dans la plage de 25 à 90% en poids d'ammoniac liquide.

13. Procédé selon l'une quelconque des revendications précédentes, **caractérisé en ce que** la partie du produit liquide évacué du réacteur qui est recyclée directement ou indirectement comme flux en boucle à l'entrée du réacteur représente 20 à 95% en poids du produit liquide total évacué du réacteur.

14. Procédé selon l'une quelconque des revendications précédentes, **caractérisé en ce que** le rapport pondéral de flux alimenté de phtalodinitrile frais + flux alimenté d'ammoniac au flux en boucle autour du réacteur d'hydrogénation se situe dans la plage de 0,05 à 5.

15. Procédé selon l'une quelconque des revendications précédentes, **caractérisé en ce que** le rapport pondéral du flux partiel recyclé du flux en boucle autour du réacteur d'hydrogénation à la somme du flux alimenté de phtalodinitrile frais et du flux alimenté d'ammoniac se situe dans la plage de 1:0,3 à 1:2.

16. Procédé selon l'une quelconque des revendications 1 à 6 ou 8 ou 15, **caractérisé en ce qu'**il s'agit, pour le flux qui est soutiré au moins partiellement comme flux partiel du flux en boucle autour du réacteur d'hydrogénation (flux b), du flux total dans le circuit en boucle et le rapport pondéral du flux partiel recyclé du produit évacué du réacteur à la somme du flux alimenté de phtalodinitrile frais et du flux alimenté d'ammoniac se situe dans la plage de 2:1 à 10:1.

17. Procédé selon l'une quelconque des revendications précédentes, **caractérisé en ce que** l'hydrogénation est réalisée à une température dans la plage de 40 à 150°C.

18. Procédé selon l'une quelconque des revendications précédentes, **caractérisé en ce que** l'hydrogénation est réalisée à une pression absolue dans la plage de 100 à 300 bars.

19. Procédé selon l'une quelconque des revendications précédentes, **caractérisé en ce que** l'hydrogénation est réalisée sur un catalyseur contenant Ni, Co et/ou Fe, comme catalyseur plein ou sur un support inerte.

20. Procédé selon l'une quelconque des revendications précédentes, **caractérisé en ce que** l'hydrogénation est réalisée sur un catalyseur plein de cobalt dopé au manganèse.

21. Procédé selon l'une quelconque des revendications précédentes, **caractérisé en ce que** le catalyseur est disposé dans un réacteur tubulaire ou dans un réacteur à faisceau tubulaire sous forme de lit fixe.

22. Procédé selon la revendication précédente, **caractérisé en ce que** le réacteur d'hydrogénation est exploité dans un mode de conduite à ruissellement.

23. Procédé selon l'une quelconque des revendications précédentes, **caractérisé en ce que** le réacteur d'hydrogénation est exploité de manière adiabatique.

24. Procédé selon l'une quelconque des revendications précédentes, **caractérisé en ce que** de la chaleur est soutirée du flux en boucle autour du réacteur d'hydrogénation dans un refroidisseur.

25. Procédé selon l'une quelconque des revendications précédentes, **caractérisé en ce qu'**on utilise une cascade de réacteurs d'hydrogénation comme réacteur d'hydrogénation.

26. Procédé selon l'une quelconque des revendications précédentes, **caractérisé en ce qu'**on réalise, après l'hydrogénation, une purification de la xylylènediamine par élimination par distillation de l'ammoniac ainsi que le cas échéant de produits secondaires à bas point d'ébullition via la tête et séparation par distillation d'impuretés à point d'ébullition élevé via le fond.

27. Procédé selon la revendication précédente, **caractérisé en ce que** la xylylènediamine est extraite avec un solvant organique après la distillation en vue d'une nouvelle purification.

28. Procédé selon la revendication précédente, **caractérisé en ce qu'**on utilise du cyclohexane ou du méthylcyclohexane pour l'extraction.
